# EUROPEAN PATENT APPLICATION

(11) **EP 1 609 779 A1**
(43) Date of publication of application: **28.12.2005**
(21) Application number: 04722465.4
(22) Date of filing: 22.03.2004
(51) Int. Cl.: C07C 403/24, C09B 61/00, C09B 67/46, A23L 1/275

(54) **PROCESS FOR PRODUCING CAROTENOID EMULSION**

(30) Priority: 28.03.2003 JP 2003089532
(71) Applicant: KURARAY CO., LTD., Kurashiki-shi, Okayama 710-8622 (JP)
(72) Inventor: MORI, Toshiki c/o Kuraray Co., Ltd., Kitakanbara-gun, Niigata 959-2653 (JP); OGATA, Toshio c/o Kuraray Co., Ltd., Kitakanbara-gun, Niigata 959-2653 (JP); SHIMAMURA, Shigetaka c/o Kuraray Co., Ltd., Kitakanbara-gun, Niigata 959-2653 (JP); TANAKA, Hiroshi c/o Kuraray Co., Ltd., Kitakanbara-gun, Niigata 959-2653 (JP); TANIGUCHI, Keita c/o Kuraray Co., Ltd., Kitakanbara-gun, Niigata 959-2653 (JP); KITAYAMA, Masahiko c/o Kuraray Co., Ltd., Kitakanbara-gun, Niigata 959-2653 (JP)
(74) Representative: Müller-Boré & Partner Patentanwälte
(86) International application number: PCT/JP2004/003880
(87) International publication number: WO 2004/087645

(57) **Abstract**

A process for producing a carotenoid emulsion **characterized by** passing a suspension of a carotenoid in toluene through a heated pipe at such a rate as to result in a residence time of 10 to 600 seconds to thereby heat the suspension to 50 to 120°C and dissolve the carotenoid, immediately mixing the resultant solution with water having a temperature of 5 to 60°C in the presence of an emulsifying agent to form an emulsion, and distilling off the toluene under vacuum. By the process, an emulsion containing a carotenoid as an active ingredient can be easily and industrially advantageously produced with satisfactory productivity while maintaining a high proportion of wholly trans molecules in the carotenoid.

## Description

### TECHNICAL FIELD

This invention relates to a process for producing a carotenoid emulsion.

### BACKGROUND ART

Carotenoids are broadly present in nature, and widely used as colorants for food, etc. utilizing the characteristic that they have yellow to red color. Some carotenoids are also known to show provitamin A activity, cancer-inhibiting effect or the like, and are a compound group drawing attention also from a pharmacological viewpoint. Many isomers are present in carotenoids based on many carbon-carbon double bonds which they possess, and when a use as colorants or a use as a physiologically active agent such as a provitamin A active agent is considered, carotenoids having a high all trans-form proportion are preferred.

Carotenoids are compounds which are in a crystalline state at ordinary temperature and have a high melting point, are insoluble in water, extremely low in solubility in organic solvents or fats and oils, and, moreover, tend to be isomerized with heat, and tend to be readily denatured with oxygen or light. Therefore, when it is intended to use carotenoids as colorants for food or physiologically active agents, they need to be processed into stable and readily utilizable forms. As one process for it, there is a process of producing an emulsion of a carotenoid by mixing a solution of the carotenoid in an organic solvent or fat or oil with water in the presence of an emulsifier.

Known processes of producing an emulsion of a carotenoid include
(1) a process which comprises emulsifying a solution of a carotenoid in a volatile water-immiscible solvent such as a halogenated hydrocarbon or carbon disulfide, into an aqueous solution of a colloid having swelling properties; and removing the volatile solvent from the resulting emulsion (see JP-A-37-12428),
(2) a process which comprises emulsifying a super-saturated solution of a carotenoid in an edible oil which is liquid at about 20 to 40°C, which solution is heated to 100 to 160 °C into an aqueous gelatin substance solution (see US-A-2,861,891),
(3) a process which comprises mixing a solution of a carotenoid in a volatile organic solvent such as a halogenated hydrocarbon with an aqueous solution containing sodium lauryl sulfate or the like, while removing the volatile organic solvent (see JP-A-51-41732 and JP-A-52-84232),
(4) a process which comprises rapidly dissolving a carotenoid, together with an edible oil of 1.5 to 20 times the mass of the carotenoid and an appropriate amount of an emulsifier, in a volatile organic solvent miscible with water at a temperature of 50 to 240°C, and then immediately mixing the resulting solution with an aqueous solution of a protective colloid at a temperature of 0 to 50°C to move the hydrophilic solvent components into the aqueous phase, and at that time changes the hydrophobic oil phase containing and dissolving the carotenoid to a fine dispersion phase (see JP-A-63-196242),
(5) a process which comprises contacting a suspension of a carotenoid in a high boiling oil with superheated steam for 30 seconds at most and emulsifying the resulting mixture in an aqueous solution of a colloid (see JP-A-3-66615),
(6) a process which comprises heating trans-form β-carotene, fats or oils and limonene to make a solution, recovering the limonene and converting the resulting fat or oil layer dissolving the trans-form β-carotene to an emulsion in the presence of an emulsifier (see JP-A-8-119933),
(7) a process which comprises feeding a suspension of a carotenoid in a water-immiscible organic solvent into a heat exchanger for a residence time of less than 5 seconds to heat the suspension to 100 to 250°C, rapidly mixing the resulting solution with an aqueous solution of easy to swell colloid at a temperature in the range of 20 to 100°C, and then removing the organic solvent (see JP-A-2000-186224),
(8) a process which comprises heating a suspension of a carotenoid in a high boiling organic liquid by passing the suspension through a conduit of 0.1 to 50 mm inside diameter heated to a temperature in the range of 120 to 700°C for a residence time of 0.05 to 5 seconds to dissolve the carotenoid, and then immediately adding the resulting solution into an aqueous solution containing an emulsifier to emulsify the solution (see JP-A-2002-302479), and
(9) a process which comprises mixing a suspension of a carotenoid in a high boiling organic liquid with a high boiling organic liquid heated to a temperature in the range of 120 to 500°C for a time of 0.05 to 10 seconds, to dissolve the carotenoid, and then immediately adding the resulting solution into an aqueous solution containing an emulsifier to emulsify the solution (see JP-A-2002-316924).

### DISCLOSURE OF INVENTION

The processes of the above (1) and (3) have a problem that although an organic solvent having comparatively high solubility on carotenoids even at ordinary temperature such as a halogenated hydrocarbon, e.g. chloroform or carbon disulfide is used, these solvents have high toxicity and severe control has recently been required on their use from the viewpoint of an environmental problem, and thus they are hard to use industrially. The process of the above (2) has a problem to be economically disadvantageous because the visible absorption spectrum of dry powder of carotenoid obtained from the resulting emulsion gets low, and for example in the case of using the dry powder as a colorant for food, the use quantity of the dry powder needs to be increased to obtain a desired value of color strength. The processes of the above (4) and (7) have the problems that since the organic solvent to be used is unnecessary for the final product, it needs to be removed and that use of a large quantity of the organic solvent is needed, which lowers productivity. The process of the above (5) has the problems that it needs expensive apparatuses because superheated steam being high temperature and high pressure is used and further that since water originating in the superheated steam, in addition to the water contained in the aqueous solution of the colloid, joins the resulting emulsion, when the production of carotenoid powder from the emulsion is intended, a large quantity of water needs to be removed. The process of the above (6), wherein limonene is used in view of inhibiting isomerization of trans-form β-carotene when the trans-form β-carotene is heated for dissolution, has the problems that the same quantity or more of the limonene as that of the fat or oil needs to be used and further that since limonene is not needed in the final product, a step to remove it is indispensable, and moreover that the total all trans-form proportion of β-carotene in the resulting emulsion is as low as on the order of 60 to 67 %. As to the processes of the above (8) and (9), large-scale ancillary facilites for generating high temperature are necessary, and, in that view, these processes have room to be improved.

Thus, the object of the invention lies in providing a process capable of producing an emulsion containing a carotenoid as an effective ingredient with the total all trans-form proportion of the carotenoid maintained high, with good productivity, conveniently, and industrially advantageously.

The present inventors have made intense researches for accomplishing the above object. As a result, we found that, by using a process of passing a suspension of a carotenoid in toluene through a heated conduit for a short time, to heat the suspension to a temperature in a particular range and dissolve the carotenoid; immediately adding the resulting solution into an aqueous solution of a temperature in a particular range containing an emulsifier to give an emulsion; and then distilling off the toluene under reduced pressure, a carotenoid emulsion not containing the unnecessary organic solvent and a large quantity of water can be produced as a final product. We further found that carotenoid powder obtained by spray drying a carotenoid emulsion obtained by such a process or by stirring the carotenoid emulsion in a nonpolar solvent to make the emulsion particles, and filtering and drying the particles can be used as colorants for food or a physiologically active agent, and completed the invention.

Namely, the present invention relates to
<1> a process for producing a carotenoid emulsion which comprises heating a suspension of the carotenoid in toluene (hereinafter merely referred to as "carotenoid suspension" ) to a temperature in the range of 50 to 120°C, by passing the suspension through a heated conduit for a residence time of the range of 10 to 600 seconds, to dissolve the carotenoid, immediately mixing the resulting solution with water of a temperature in the range of 5 to 60°C in the presence of an emulsifier to emulsify the solution, and then distilling off the toluene under reduced pressure, and
<2> carotenoid powder obtained by spray drying the resulting carotenoid emulsion obtained in <1>, or by stirring the carotenoid emulsion in a nonpolar solvent to make the emulsion particles, and filtering and drying the particles.

### BRIEF DESCRIPTION OF THE DRAWING

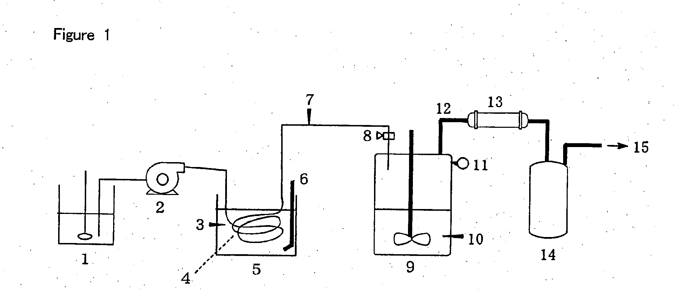

Figure 1 shows an embodiment of the production process of the invention. In Figure 1 the symbols have the following meanings, respectively.
1 Feed vessel (equipped with an agitator)
2 Metering pump
3, 7, 10 Thermometer
4 Conduit
5 Vessel containing a heat medium
6 Heating apparatus
8 Cock
9 Preparation vessel (equipped with a homogenizer)
11 Manometer
12 Piping for distilling-off
13 Condenser
14 Receiver
15 Vacuum pump

### BEST MODE FOR CARRYING OUT THE INVENTION

As carotenoids used in the invention, there can be exemplified α-carotene, β-carotene, canthaxanthin, astaxanthin, apocarotenal, citranaxanthin, cryptoxanthin, lycopene, zeaxanthin, etc. These carotenoids can be used alone or as a mixture of two or more. As to the form of carotenoids used, in view of smoothly carrying out the dissolution of the carotenoids in toluene, it is preferred that the carotenoids are crystals having a particle size of 50 µm or less and it is further preferred that the carotenoids are crystals having a particle size of 15 µm or less.

A solvent used to obtain a carotenoid suspension in the invention is toluene. Toluene has an advantage that the solubility of carotenoids in it is largely different between at ordinary temperature and at the time of heating.

Carotenoids are sensitive to oxygen, and therefore it is preferred to add an antioxidant in preparation of a carotenoid suspension. As the antioxidant, there can, for example, be mentioned t-butylhydroxyanisole, t-butylhydroxytoluene, vitamin E, ethoxyquin, etc., and vitamin E is particularly preferred. The antioxidant can be mixed with toluene at any proportion and used, but it is, usually, preferred to use the antioxidant in the range of 10 times or less the mass of the carotenoid used.

There is no particular limitation on the proportion of the carotenoid to toluene in preparation of a carotenoid suspension, but, in view of the solubility of the carotenoid, productivity and the like, the use quantity of carotenoid is preferably in the range of 0.1 to 10 % by mass, more preferably in the range of 0.5 to 5 % by mass, based on the toluene.

According to the invention, first, the carotenoid suspension is heated in a short time by passing the suspension through a heated conduit, to dissolve the carotenoid.

The heating temperature and heating time of the carotenoid suspension may vary depending on the kind of the carotenoid composing the suspension, the use proportion of the carotenoid to toluene, the passing quantity and passing rate of the suspension, etc., but in view of inhibiting isomerization of the carotenoid with heat, it is necessary that the heating temperature is in the range of 50 to 120°C and the heating time is in the range of 10 to 600 seconds, and it is preferred that the heating temperature is in the range of 60 to 110°C and the heating time is in the range of 10 to 30 seconds.

The heating temperature of the conduit may vary depending on the kind of the carotenoid composing the suspension to be passed, the use proportion of the carotenoid to toluene, the passing quantity and passing rate of the suspension, etc., but, usually, the heating temperature is preferably in the range of 50 to 200°C and more preferably in the range of 70 to 150°C.

As means for heating the conduit, ordinary heating means can be adopted such as a method to use a gas burner, an electric heater, electromagnetic induction or the like; a method to heat the conduit by using an organic heating medium such as a usual heating medium oil or an inorganic heating medium such as HTS (Heat Transfer Salt: mixture of sodium nitrite, sodium nitrate and potassium nitrate); and a method to use a high temperature gas such as steam or heated air.

The material of the conduit is not particularly limited so long as it is usable in the process of the invention, and includes, for example metals such as iron, stainless steel and titanium, glasses, etc. As to the shape of the conduit, any shape can be adopted, and examples thereof are linear conduits, conduits of a coil shape, etc. Further, in view of further improving productivity, it is also possible to use plural conduits with parallel connection.

In the process of the invention, the inside diameter of the conduit to be used is not particularly limited, but, usually, is preferably in the range of 0.1 to 500 mm and more preferably in the range of 0.5 to 100 mm. When a thin conduit of which inside diameter is less than 0.1 mm is used, clogging is liable to occur in the conduit when the carotenoid suspension is passed, and on the other hand, when a conduit having an inside diameter of more than 500 mm is used, it gets very hard to heat the carotenoid suspension, in a short time, to such a temperature that the carotenoid dissolves in toluene.

As to the thickness of the conduit, there is no particular limitation, but in view of efficiently supplying quantity of heat to the carotenoid suspension and in view of withstanding the pressure given to the conduit when the suspension is passed, the thickness of the conduit is, usually, preferably in the order of 1/10 to 10 times the inside diameter of the conduit, more preferably in the order of 1/5 to 5 times the inside diameter.

The length of the conduit of the part heated within the prescribed range may vary depending on the kind of the carotenoid composing the carotenoid suspension to be passed, the use proportion of the carotenoid to toluene, the passing quantity and passing rate of the suspension, etc., but, in view of supplying enough heat quantity to dissolve the carotenoid in toluene within the time for the carotenoid suspension to pass through the conduit and in view of inhibiting the isomerization of the carotenoid with heat as completely as possible during the time, the length of the conduit of the part is, usually, preferably in the range of 0.1 to 20 m, more preferably in the range of 0.5 to 10 m.

As means for transferring the carotenoid suspension to the conduit of the part heated to the prescribed temperature, means usually used when liquid is transferred, such as metering pumps and compressed gases, can be used. The amount of the carotenoid suspension to be transferred may vary depending on the kind of the carotenoid composing the suspension to be passed, the use proportion of the carotenoid to toluene, the heating temperature of the conduit, and the heating time of the suspension, etc., but the amount is, usually, preferably in the range of 0.1 to 10 kg/minute.

There is no particular limitation on the size and length of the conduit up to introduction of the carotenoid suspension into the inlet of the heated conduit part. On the other hand, the length of the conduit from the outlet of the heated conduit part to the emulsifying apparatus, is, usually, preferably in the range of 0.01 to 20 m in view of inhibiting the isomerization of the carotenoid with heat, etc.

Advantageously, it is, for example, possible to use tubes of the same material and the same diameters as the conduit from the feed vessel charged with the carotenoid suspension to the introduction part of the heated conduit, the heated conduit and the conduit from the heated conduit to the emulsifying apparatus.

As mentioned above, by passing the carotenoid suspension through the heated conduit, a solution wherein the carotenoid is in a state of being dissolved in toluene (hereinafter, referred to as "carotenoid solution" ) can be prepared, and the solution is then immediately subjected to a step of mixing with water, in the presence of an emulsifier, to emulsify the solution.

As to the emulsifier, there is no particular limitation so long as it is capable of emulsifying the carotenoid solution and water, and there can, for example, be mentioned fatty acid esters of ascorbic acid such as ascorbic acid palmitate and ascorbic acid monooleate; sucrose fatty acid esters such as sucrose palmitate and sucrose monooleate; sorbitan fatty acid esters such as sorbitan palmitate and sorbitan monooleate; polyglycerol fatty acid esters such as polyglycerol palmitate and polyglycerol monooleate; etc. Among them, it is particularly preferred to use fatty acid esters of ascorbic acid such as ascorbic acid palmitate. The emulsifier may be added into the water in advance, or may be mixed into the carotenoid suspension or the carotenoid solution. When a fatty acid ester of ascorbic acid is added into the water in advance, a basic alkali metal compound such as sodium hydroxide or sodium carbonate may be added in order to make the dissolution of the fatty acid ester of ascorbic acid easy.

The use amounts of the emulsifier and the water are not particularly limited so long as the carotenoid solution can be emulsified, namely a stable O/W emulsion can be formed. But, usually, the use amount of the emulsifier is preferably in the range of 0.05 to 15 % by mass, more preferably in the range of 0.1 to 5 % by mass, based on the water used. The use amount of the water may vary depending on the kind of the carotenoid, the use proportion of the carotenoid to toluene, etc., but is preferably in the range of 1 to 2,000 times the mass of the carotenoid, more preferably in the range of 10 to 500 times the mass of the carotenoid.

In view of improving the stability of the carotenoid emulsion to be produced, gelatin, sugars, gum arabic, starches or the like may be added into the water. As the sugars, there can be mentioned sucrose, invert sugar, etc.

At the time when the carotenoid solution is mixed with water in the presence of the emulsifier to emulsify the solution, the temperature of the water is needed to be in the range of 5 to 60°C, and is preferably in the range of 30 to 50°C. When the temperature of the water is less than 5°C, the emulsion state gets bad, and when it is more than 60°C, the quality of the carotenoid solution gets bad.

The step to mix the carotenoid solution with water, in the presence of an emulsifier, to emulsify the solution can be carried out, for example, by a process of charging an aqueous solution of the emulsifier in advance into a vessel equipped with an agitation-type emulsifier, and then adding the carotenoid solution thereinto intermittently or continuously to emulsify the solution; a process of charging water in advance into a vessel equipped with an agitation-type emulsifier, and then adding thereinto the carotenoid solution containing the emulsifier intermittently or continuously to emulsify the solution; a process of introducing the carotenoid solution and the aqueous solution of the emulsifier together into a line mixer to emulsify the solution; a process of introducing the carotenoid solution containing the emulsifier, and water together into a line mixer to emulsify the solution; etc.

Then, a step to distill off the toluene under reduced pressure, from an emulsion of the carotenoid containing toluene, obtained by mixing the carotenoid solution with water in the presence of the emulsifier according to a process as mentioned above, is carried out.

The temperature at which the toluene is distilled off is preferably in the range of 5 to 60°C, more preferably in the range of 30 to 50°C. As to the degree of reduced pressure in the distilling-off of the toluene, such a degree of reduced pressure can appropriately be selected that the distilling-off of the toluene is possible within the above temperature range. The distilling-off of the toluene is carried out until toluene comes not to exist in the emulsion of the carotenoid. Since toluene is distilled off as an azeotrope with water, it is also possible to separate the distilled water from the toluene and bring it back to the carotenoid emulsion.

It is also possible to carry out the step of mixing the carotenoid solution with water in the presence of the emulsifier to emulsify the solution and the step of distilling off toluene under reduced pressure from the carotenoid emulsion containing toluene, at the same time.

The process of the invention is a series of operations comprising an operation of heating a carotenoid suspension to a temperature in the range of 50 to 120°C by passing the suspension through a heated conduit for a residence time in the range of 10 to 600 seconds, to dissolve the carotenoid; an operation of immediately mixing the resulting solution with water of a temperature in the range of 5 to 60°C, in the presence of an emulsifier, to emulsify the solution; and an operation of distilling off toluene under reduced pressure, and a carotenoid emulsion can be obtained with such convenient operations.

The obtained carotenoid emulsion can be used as it is for a use such as a colorant for food or a feed additive. Further, by spray drying the carotenoid emulsion, or by stirring the carotenoid emulsion in a nonpolar solvent such as hexane, toluene or a paraffin, to convert the carotenoid emulsion to particles, and filtering and drying the particles, powder containing the carotenoid can be obtained. The carotenoid powder can be used for uses such as a colorant for food and a physiologically active agent.

### EXAMPLES

The present invention is specifically described below through examples, but the invention is not limited at all by these examples.

An embodiment of the production process of the present invention is shown in Figure 1. A carotenoid suspension and an emulsifier are charged into Feed vessel 1. The carotenoid suspension containing the emulsifier is fed from Feed vessel 1 into Conduit 4 immersed in a heating medium in Vessel 5 heated by Heating apparatus 6, via Metering pump 2, and thereby the suspension becomes, in the heated Conduit 4, such a solution that the carotenoid is in a state of being dissolved in the toluene. This solution is immediately intermittently or continuously fed into Preparation vessel 9. Vacuum pump 15 can reduce the pressure up to Preparation vessel 9, and toluene, distilled off under reduced pressure from the carotenoid emulsion containing toluene prepared in Preparation vessel 9, is recovered into Receiver 14 via Piping for distilling-off 12 and Condenser 13.

### Example 1 Production of β-carotene emulsion

In Figure 1, as Conduit 4 was used such a coil-shaped stainless steel conduit that the inside diameter is 2 mm, the outside diameter is 3 mm and the length is 4m. This Conduit 4 was put in Vessel 5 containing a heat medium and heated to 90°C.

700 g of water and 35 g of sucrose were put in Preparation vessel 9, and 30 g of gelatin was put therein, then the mixture was stirred at 40°C to dissolve the gelatin, and then 15 g of 1N aqueous sodium hydroxide solution was added so that the pH became 7 to 8. Separately, 28 g of β-carotene, 1,400 g of toluene, 5.6 g of ascorbic acid palmitate and 5.6 g of vitamin E were put in Feed vessel 1 and stirred mildly. The pressure from Preparation vessel 9 to Receiver 14 was reduced to 13.3 kPa.s(100 mmHg) by Vacuum pump 15, the homogenizer of Preparation vessel 9 was operated at 9,000 r.p.m., and the suspension in Feed vessel 1 was transferred to Preparation vessel 9 at a flow rate of 18 g/minute by Metering pump 2. At that time, the residence time of the feed liquid in the heated Cnduit 4 was about 36 seconds, the temperature of the β-carotene solution at the outlet of the heated Conduit 4 was 86°C, and there was almost no crystals of β-carotene remaining undissolved.

The feed of the suspension of Feed vessel 1 was continued while toluene and water distilling in a short time after the start of feed were received in Receiver 14, but, three times during the operation, the feed of the suspension was temporarily stopped, and after the liquid quantity in Preparation vessel 9 became appropriate, the feed was restarted. Water collected in Receiver 14 was removed at times, and after separation from toluene, brought back to Preparation vessel 9. The β-carotene suspension in Feed vessel 1 was used up in 7 hours after the start of feed, but the distillation operation was continued, and, after additional 5 hours, after confirming that the quantity of toluene in Preparation vessel 9 became 0.1 % or less, holding the reduced pressure from Preparation vessel 9 to Receiver 14 was stopped and the stirring by the homogenizer was stopped. When the resulting orange emulsion (760 g) was analyzed, this emulsion contained 3.6 % of β-carotene and its total all trans-form proportion was 90 %.

### Example 2 Production of canthaxanthin emulsion

The same apparatus as in Example 1 was used, and the temperature of the heat medium was made to 95°C. 700 g of water and 35 g of sucrose were put in Preparation vessel 9, and 30 g of gelatin was put therein, then the mixture was stirred at 40°C to dissolve the gelatin, and then 15 g of 1N aqueous sodium hydroxide solution was added so that the pH became 7 to 8. Separately, 28 g of canthaxanthin, 1,400 g of toluene, 5.6 g of ascorbic acid palmitate and 5.6 g of vitamin E were put in Feed vessel 1 and stirred mildly. The pressure from Preparation vessel 9 to Receiver 14 was reduced to 13.3 kPa (100 mmHg) by Vacuum pump 15, the homogenizer of Preparation vessel 9 was operated at 9,000 r.p.m., and the suspension in Feed vessel 1 was transferred to Preparation vessel 9 at a flow rate of 16 g/minute by Metering pump 2. At that time, the residence time of the feed liquid in the heated Conduit 4 was about 41 seconds, the temperature of the canthaxanthin solution at the outlet of the heated Conduit 4 was 90°C, and there was almost no crystals of canthaxanthin remaining undissolved. Feed and distillation operations were carried out in the same way as in Example 1, and when the resulting orange emulsion (763 g) was analyzed, this emulsion contained 3.5 % of canthaxanthin and its total all trans-form proportion was 85 %.

### Example 3 Production of astaxanthin emulsion

The same apparatus as in Example 1 was used, and the temperature of the heat medium was made to 108°C. 700 g of water and 35 g of sucrose were put in Preparation vessel 9, and 30 g of gelatin was put therein, then the mixture was stirred at 40°C to dissolve the gelatin, and then 15 g of 1N aqueous sodium hydroxide solution was added so that the pH became 7 to 8. Separately, 28 g of astaxanthin, 2,000 g of toluene, 5.6 g of ascorbic acid palmitate and 5.6 g of vitamin E were put in Feed vessel 1 and stirred mildly. The pressure from Preparation vessel 9 to Receiver 14 was reduced to 13.3 kPa (100 mmHg) by Vacuum pump 15, the homogenizer of Preparation vessel 9 was operated at 9,000 r.p.m., and the suspension in Feed vessel 1 was transferred to Preparation vessel 9 at a flow rate of 16 g/minute by Metering pump 2. At that time, the residence time of the feed liquid in the heated Conduit 4 was about 45 seconds, the temperature of the astaxanthin solution at the outlet of the heated Conduit 4 was 103°C, and there was almost no crystals of astaxanthin remaining undissolved. Feed and distillation operations were carried out in the same way as in Example 1, and when the resulting orange emulsion (736 g) was analyzed, this emulsion contained 3.7 % of astaxanthin and its total all trans-form proportion was 84 %.

### Comparative example 1 Production of canthaxanthin emulsion (under the condition of not using a conduit for heating)

700 g of water and 35 g of sucrose were put in Preparation vessel 9 in Figure 1, and 30 g of gelatin was put therein, then the mixture was stirred at 40°C to dissolve the gelatin, then 15 g of 1N aqueous sodium hydroxide solution was added so that the pH became 7 to 8, and the homogenizer of Preparation vessel 9 was operated at 9,000 r.p.m.

On the other hand, 28 g of canthaxanthin, 1,400 g of toluene, 5.6 g of ascorbic acid palmitate and 5.6 g of vitamin E were put in a three-necked flask of content volume 3,000 ml equipped with a reflux condenser, and the mixture was heated under stirring with a heat medium bath of 130°C, and held under a condition of reflux of toluene for 15 minutes to make the canthaxanthin dissolve. Then, the whole quantity of the resulting toluene solution of canthaxanthin was immediately poured all at once into Preparation vessel 9. In this connection, there was almost no crystals of canthaxanthin remaining undissolved in the toluene solution poured. After the stirring with the homogenizer was continued for 30 minutes, the same operation for distillation of toluene and water as in Example 1 was carried out, and when the resulting orange emulsion (780 g) was analyzed, this emulsion contained 3.5 % of canthaxanthin and its total all trans-form proportion was 38 %.

### INDUSTRIAL APPLICABILITY

Carotenoids which are broadly used as coloring agents for food and use of which from a pharmacological viewpoint is also considered, are needed, in view of solubility and stability, to be processed into a stable and easy to utilize form, and as one of such processes, there is a process of producing a carotenoid emulsion.

According to the present invention, an emulsion containing a carotenoid as an effective ingredient can be produced with the total all trans-form proportion of the carotenoid maintained high, with good productivity, conveniently, and industrially advantageously.

## Claims

1. A process for producing a carotenoid emulsion which comprises heating a suspension of the carotenoid in toluene to a temperature in the range of 50 to 120°C, by passing the suspension through a heated conduit for a residence time in the range of 10 to 600 seconds, to dissolve the carotenoid, immediately mixing the resulting solution with water of a temperature in the range of 5 to 60°C in the presence of an emulsifier to emulsify the solution and then distilling off the toluene under reduced pressure.

2. The process according to claim 1, wherein the particle size of the carotenoid is 50 µm or less.

3. The process according to claim 1 or 2, wherein the use quantity of the carotenoid to the toluene is in the range of 0.1 to 10 % by mass.

4. The process according to any of claims 1 to 3, wherein the suspension of the carotenoid contains an antioxidant.

5. The process according to any of claims 1 to 4, wherein the course of the solution from the outlet of the heated conduit to immediately before the mixing with the water is also formed with a conduit.

6. The process according to any of claims 1 to 5, wherein the inside diameter of the conduit is in the range of 0.1 to 500 mm.

7. The process according to any of claims 1 to 6, wherein the thickness of the conduit is in the range of 0.1 to 10 times the inside diameter of the conduit.

8. The process according to any of claims 1 to 7, wherein the length of the heated conduit is in the range of 0.1 to 20 m.

9. The process according to any of claims 5 to 8, wherein the length of the conduit from the outlet of the heated conduit to immediately before the mixing with the water is in the range of 0.01 to 20 m.

10. The process according to any of claims 1 to 9, wherein the emulsifier is at least one selected from a fatty acid ester of ascorbic acid, a sucrose fatty acid ester, a sorbitan fatty acid ester and a polyglycerol fatty acid ester.

11. The process according to any of claims 1 to 10, wherein the use amount of the emulsifier is in the range of 0.1 to 5 % by mass of the water used.

12. The process according to any of claims 1 to 11, wherein the use amount of the water is in the range of 1 to 2,000 times the mass of the carotenoid.

13. The process according to any of claims 1 to 12, wherein at least one selected from gelatin, sugar, gum arabic and starch is added into the water.

14. Carotenoid powder obtained by spray drying the carotenoid emulsion obtained by the process according to any of claims 1 to 13, or by stirring the carotenoid emulsion in a nonpolar solvent to make the emulsion particles, and filtering and drying the particles.
